(19) Europäisches Patentamt
European Patent Office
Office européen des brevets

(11) **EP 3 060 334 B1**

(12) **EUROPÄISCHE PATENTSCHRIFT**

(45) Veröffentlichungstag und Bekanntmachung des Hinweises auf die Patenterteilung:
**18.12.2019 Patentblatt 2019/51**

(21) Anmeldenummer: **14772126.0**

(22) Anmeldetag: **23.09.2014**

(51) Int Cl.:
*B01J 19/24* *(2006.01)*     *B01J 19/26* *(2006.01)*
*B01J 10/00* *(2006.01)*

(86) Internationale Anmeldenummer:
**PCT/EP2014/070194**

(87) Internationale Veröffentlichungsnummer:
**WO 2015/058919 (30.04.2015 Gazette 2015/17)**

(54) **STRAHLSCHLAUFENREAKTOR MIT NANOFILTRATION UND GASSEPARATOR**

JET LOOP REACTOR WITH NANOFILTRATION AND GAS SEPARATOR

RÉACTEUR À BOUCLE À RAYONNEMENT COMPRENANT UNE NANOFILTRATION ET UN SÉPARATEUR DE GAZ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **25.10.2013 DE 102013221708**

(43) Veröffentlichungstag der Anmeldung:
**31.08.2016 Patentblatt 2016/35**

(73) Patentinhaber: **Evonik Operations GmbH**
**45128 Essen (DE)**

(72) Erfinder:
• **FRANKE, Robert**
**45772 Marl (DE)**
• **HAMERS, Bart**
**NL-5961 RM Horst (NL)**

(74) Vertreter: **Evonik Patent Association**
**c/o Evonik Industries AG**
**IP Management**
**Bau 1042A/PB 15**
**Paul-Baumann-Straße 1**
**45772 Marl (DE)**

(56) Entgegenhaltungen:
DE-A1- 19 632 600     DE-A1-102005 046 250
DE-A1-102006 003 618     DE-A1-102009 016 652
DE-A1-102012 202 779

**Beschreibung**

Die Erfindung betrifft eine Vorrichtung für die kontinuierliche, homogenkatalytische Umsetzung einer Flüssigkeit mit einem Gas und gegebenenfalls einem weiteren Fluid, wobei die Vorrichtung mindestens einen Strahlschlaufenreaktor mit einem von mindestens einer Pumpe getriebenen, äußeren Flüssigkeitsumlauf umfasst, und wobei die Vorrichtung mindestens eine den Homogenkatalysator bevorzugt zurückhaltende Membrantrenneinheit aufweist, welche in dem äußeren Flüssigkeitsumlauf des Strahlschlaufenreaktors angeordnet ist.

Eine Flüssigkeit ist ein im Wesentlichen inkompressibles, fließfähiges Medium. Ein Gas ist ein kompressibles, fließfähiges Medium. Ein Fluid ist eine Flüssigkeit oder ein Gas. Ein zweiphasiges Gemisch aus einer homogen verteilten flüssigen Phase und eine dispers darin verteilte Gasphase ist ebenfalls ein Fluid im Sinne dieser Erfindung. Aufgrund des Gasanteils sind derartige Fluide im geringen Umfang kompressibel.

Im Rahmen der vorliegenden Erfindung wird unter zugeführter Flüssigkeit ein Stoff oder Stoffgemisch verstanden, der/das in der Apparatur unter Reaktionsbedingungen im flüssigen Aggregatzustand vorliegt und mindestens einen Reaktanden aufweist. Unter Gas wird ein reines Gas oder ein Gasgemisch, das zumindest einen Reaktanden und gegebenenfalls ein Inertgas aufweist, verstanden. Ein Beispiel für ein Gas, das zwei Reaktanden aufweist, ist das aus Wasserstoff und Kohlenmonoxid bestehende Synthesegas, das beispielsweise bei Hydroformylierungen eingesetzt wird.

Bei einem Strahlschlaufenreaktor (engl.: jet loop reactor) im Sinne der Erfindung handelt es sich um eine Vorrichtung für die kontinuierliche Umsetzung einer Flüssigkeit und mindestens eines weiteren Fluids, bei der die Flüssigkeit unter Druck durch eine Düse in einen Reaktionsraum eintritt, diesen längs einer Hauptstromrichtung durchströmt, an dem gegenüber der Düse gelegenen Ende des Reaktionsraums umgelenkt wird, entgegen der Hauptstromrichtung zurückströmt und erneut in Hauptstromrichtung beschleunigt wird, sodass sich innerhalb des Reaktorraums ein interner Flüssigkeitskreislauf (Schlaufe) einstellt. Das zweite Fluid wird von der Flüssigkeitsströmung mitgerissen und reagiert auf dem Weg entlang der Schlaufe. Die Flüssigkeit dient mithin als Treibstrahlmedium. Zum Eintrag der kinetischen Energie in die Flüssigkeit ist dem Reaktionsraum ein äußerer Flüssigkeitsumlauf zugeordnet, in welchem ein Teil der Flüssigkeit außerhalb des Reaktionsraums im Kreislauf geführt wird. Innerhalb des äußeren Flüssigkeitsumlaufs ist eine Pumpe vorgesehen, welche dem Flüssigkeitsstrom die für die Etablierung der Schleifenströmung innerhalb des Reaktors notwendige kinetische Energie aufprägt. Die Düse wird entsprechend aus dem äußeren Kreislauf gespeist.

Eine gute Einführung in die Technik der Strahlschlaufenreaktoren bietet:

P. Zehner, M. Krause: "Bubble Columns", Ullmann's Encyclopedia of Industrial Chemistry, Electronic Release, 7th ed., Chapter 4, Wiley-VCH, Weinheim [2005].

Bei der Hydroformylierung (auch Oxo-Reaktion genannt) werden Kohlenwasserstoffe mit olefinischen Doppelbindungen (Alkene) mit Synthesegas (Gasgemisch aus Wasserstoff und Kohlenmonoxid) zu Aldehyden und/oder Alkoholen umgesetzt.

Grundlegende Einführungen in die Hydroformylierung bieten: Falbe, Jürgen: New Syntheses with Carbon Monoxide. Springer Verlag 1980, Berlin, Heidelberg, New York und Pruett, Roy L.: Hydroformylation. Advances in Organometallic Chemistry Vol. 17, Pages 1-60, 1979.

Eine gute Übersicht über den Stand der Hydroformylierung von Olefinen findet sich in

B. Cornils, W. A. Herrmann, "Applied Homogeneous Catalysis with Organometallic Compounds", Vol. 1 & 2, VCH, Weinheim, New York, 1996 sowie in R. Franke, D. Selent, A. Börner, "Applied Hydroformylation", Chem. Rev., 2012, DOI:10.1021/cr3001803.

Die Hydroformylierung dient zur Herstellung von höheren Aldehyden. Höhere Aldehyde, insbesondere solche mit 3 bis 25 Kohlenstoffatome, werden zum Beispiel als Synthesevorstufen, zur Herstellung von Carbonsäuren und als Riechstoffe genutzt. Technisch werden sie oft durch katalytische Hydrierung in die entsprechenden Alkohole überführt, die wiederum für die Herstellung von Weichmachern und Detergentien dienen. Aufgrund der großtechnischen Bedeutung der Hydroformylierungsprodukte wird die Oxo-Reaktion im industriellen Maßstab durchgeführt.

In der großtechnischen Hydroformylierung werden heute phosphororganische Metall-Komplexkatalysatoren auf Cobalt- oder Rhodiumbasis verwendet. Die Katalysatoren werden homogen in der flüssigen Hydroformylierungsmischung gelöst. Im Rahmen der Ausscheidung des Zielprodukts (der Aldehyde) aus dem Hydroformylierungsgemisch ist auch der Homogenkatalysator aus dem Hydroformylierungsgemisch schonend abzutrennen, da der Komplexkatalysator vergleichsweise empfindlich auf Zustandsänderungen reagiert und seine Aktivität verlieren könnte.

Traditionell wird der Katalysator destillativ aus den Hydroformylierungsgemisch abgeschieden. Um die Gefahr der Desaktivierung zu senken und den Energieverbrauch des Prozesses zu senken gibt es in jüngerer Zeit Bestrebungen, den homogen gelösten Katalysator mit Hilfe von Membrantechnologie (Nanofiltration) aus dem Hydroformylierungsgemisch abzutrennen. Eine hervorragende Einführung in die Membrantechnologie bietet:

Melin / Rautenbach: Membranverfahren. Grundlagen der Modul- und Anlagenauslegung. Springer, Berlin Heidelberg 2004.

Die Grundlagen der Membrangestützten, organophilen Nanofiltration zur Abscheidung von homogen gelösten Katalysatorkomplexen aus Hydroformylierungsmischungen beschreiben Priske, M. et al.: Reaction integrated separation of homogeneous catalysts in the hydroformylation of higher olefins by means of organophilic nanofiltration. Journal of Membrane Science, Volume 360, Issues 1-2, 15 September 2010, Pages 77-83; doi:10.1016/j.memsci.2010.05.002.

Bei der Membranfiltration von Reaktoraustragen von Hydroformylierungen stellt das häufig im flüssigen Reaktoraustrag gelöste oder ungelöste Synthesegas eine Besonderheit dar: Die Hydroformylierung ist eine zweiphasige Reaktion, Wasserstoff und Kohlenmonoxid bilden die Gasphase, die Alkene, Aldehyde und Alkohole die flüssige Phase, in welcher der Katalysator feststofffrei gelöst ist. Entsprechend dem Lösungsgleichgewicht im Reaktor ist auch ein Teil des Synthesegases in der flüssigen Reaktorphase gelöst und wird mit dem Reaktoraustrag entnommen. Solange das Synthesegas in dem Reaktoraustrag während der Membranfiltration gelöst bleibt, ist die Membranfiltration insoweit unproblematisch. Falls der flüssige Reaktoraustag jedoch von einer Gasphase begleitet wird oder wenn bei Entspannung an der Membran eine Gasphase ausperlt, vermögen die Gasblasen die Membran mechanisch zu schädigen. Für Schäden durch Gasblasen sind insbesondere Polymer-Membranen anfällig.

Ein weiteres Problem durch ausgasendes Synthesegas ist der Verlust an Kohlenmonoxid: Insbesondere in der Rh-katalysierten Hydroformylierung übt der CO-Partialdruck einen entscheidenden Einfluss auf die Aktivität und Stabilität des Katalysatorkomplexes auf. Um bei der Membranabtrennung von homogen gelösten Komplexkatalysatoren aus dem Reaktionsaustrag einer Hydroformylierung den Verlust von Aktivität zu vermeiden, schlägt EP1931472B1 vor, an allen drei Anschlüssen der Membrantrenneinheit (Feed, Retentat, Permeat) einen CO-Mindestpartialdruck sicherzustellen.

WO2010023018A1 zeigt zwei parallel miteinander verschaltete Strahlschlaufenreaktoren mit einem gemeinsamen äußeren Flüssigkeitsumlauf. Die Strahlschlaufenreaktoren werden bei der Hydroformylierung mit homogen gelösten Katalysatoren eingesetzt. Die Abscheidung des Katalysators wird nicht thematisiert.

Janssen, M., Wilting, J., Müller, C. and Vogt, D. (2010), Continuous Rhodium-Catalyzed Hydroformylation of 1-Octene with Polyhedral Oligomeric Silsesquioxanes (POSS) Enlarged Triphenylphosphine. Angewandte Chemie International Edition, 49: 7738-7741. doi: 10.1002/anie.201001926 beschreiben die Durchführung einer homogen katalysierten Hydroformylierung in einem speziellen Sprühnebelreaktor, welcher über zwei äußere, in einer Querstromkammer miteinander kontaktierte Flüssigkeitsumläufe verfügt. In einem ersten Umlauf wird der flüssige Reaktoraustrag mit darin gelöstem Synthesegas aus dem Reaktor abgezogen und mittels einer Flügelzellenpumpe zirkuliert. In einer Querstromkammer wird der Reaktoraustrag in zwei Teilströme geteilt: Ein erster Teilstrom enthaltend den flüssigen Reaktoraustrag mit gelöstem Synthesegas als Synthesegas in Gasphase wird entlang des ersten Umlaufes zurück in den Reaktor geführt. Ein zweiter, rein flüssiger Teilstrom wird mittels einer Pumpe über eine keramische Membrantrenneinheit gefördert. Dort wird das Zielprodukt als Permeat abgezogen, das katalysatorhaltige Retentat über den zweiten Umlaufstrang zurück zur Querstromkammer geleitet und dort mit dem ersten Flüssigkeitsumlauf vermengt. Der Vorteil dieser Vorrichtung ist darin zu sehen, dass der Reaktoraustrag innerhalb der Querstromkammer entgast wird und somit etwaig ausfallende Gasphasen im ersten Umlauf verbleiben. Die besonderen Strömungsverhältnisse der Querstromkammer begünstigen nämlich einen Abzug der Gasblasen in den Rücklauf des ersten Kreises. Der zweite Flüssigkeitsumlauf, in welchem die Membran angeordnet ist, bleibt mithin gasfrei (das bedeutet, $H_2$ und CO bleiben in der Flüssigkeit gelöst). Der Nachteil dieses Labor-Apparats ist jedoch sein vergleichsweise komplizierter Aufbau, der Bedarf an zwei Pumpen und der große strömungsdynamische Energieverlust in der Querstromkammer: Hydroformylierungen im industriellen Maßstab lassen sich in dieser Vorrichtung kaum wirtschaftlich durchführen.

Ein Verfahren und eine Vorrichtung zur Hydroformylierung, die eine externe Rückführung eines einen homogenen Katalysator enthaltenden Produktstroms in den Reaktor umfassen, sind aus DE 10 2012 202779 bekannt.

Eine Vorrichtung der eingangs genannten Gattung ist aus WO2013/034690A1 bekannt. Hier ist die Membrantrenneinheit im äußeren Flüssigkeitsumlauf des Strahlschlaufenreaktors angeordnet. Nachteil dieser Vorrichtung ist, dass das über den äußeren Flüssigkeitsumlauf geführte Fluid neben den flüssigen Anteilen auch Gasanteile - in einer flüssigen Phase gelöst oder in einer Gasphase befindlich - transportiert. Deshalb ist das Volumen, welches durch die Membrantrenneinheit geführt werden muss, vergleichsweise groß. Der große Feedvolumenstrom für die Membrantrenneinheit erfordert eine große Membranfläche und mithin höhere Investitionskosten für die Errichtung der Anlage. Darüber hinaus sind einige Membranmaterialien gar nicht in so großen Mengen verfügbar, dass sich daraus Membrantrenneinheiten mit großen Membranflächen wirtschaftlich aufbauen ließen.

In Hinblick auf diesen Stand der Technik liegt der Erfindung die Aufgabe zu Grunde, die Kosten der Vorrichtung zu reduzieren.

Dies gelingt überraschend durch das Vorsehen eines zusätzlichen Apparats, nämlich einen Gasseparator, der im äußeren Flüssigkeitsumlauf des Strahlschlaufenreaktors angeordnet und dafür eingerichtet ist, Gas aus dem äußeren Flüssigkeitsumlauf abzuscheiden und in den Strahlschlaufenreaktor zurückzuführen.

Gegenstand der Erfindung ist daher eine Vorrichtung für die kontinuierliche, homogenkatalytische Umsetzung einer Flüssigkeit mit einem Gas und gegebenenfalls einem weiteren Fluid, wobei die Vorrichtung mindestens einen Strahlschlaufenreaktor mit einem von mindestens einer Pumpe getriebenen, äußeren Flüssigkeitsumlauf umfasst, wobei die Vorrichtung mindestens eine den Homogenkatalysator bevorzugt zurückhaltende Membrantrenneinheit aufweist, welche in dem äußeren Flüssigkeitsumlauf des Strahlschlaufenreaktors angeordnet ist, und wobei im äußeren Flüssigkeitsumlauf des Strahlschlaufenreaktors ein Gasseparator angeordnet ist, welcher dafür eingerichtet ist, Gas aus dem äußeren Flüssigkeitsumlauf abzuscheiden und in den Strahlschlaufenreaktor zurückzuführen.

Die Erfindung beruht auf der Erkenntnis, dass sich mittels des Gasseparators Gasanteile aus dem äußeren Flüssigkeitsumlauf entfernen lassen, bevor dieser auf die Membran gegeben wird. Ein Gasseparator kann aus dem im Flüssigkeitsumlauf geführten Fluid sowohl Gas abscheiden, welches in einer flüssigen Phase gelöst ist, als auch Gas, welches eine eigene Gasphase (Blasen) bildet. Das Entgasen im Gasseparator verkleinert den Feedvolumenstrom der Membrantrenneinheit, wodurch sich auch die Membranfläche und damit die Kosten der Membrantrenneinheit reduzieren lassen. Da ein Gasseparator ein Standard-Apparat darstellt, der vergleichsweise günstig und einfach verfügbar ist, ist die Kombination aus einer Membrantrenneinheit mit vorgeschaltetem Gasseparator und verkleinerter Membranfläche kostengünstiger als eine herkömmliche Vorrichtung ohne Gasseparator, die jedoch eine größere Membranfläche erfordert. Erfindungsgemäß ist die Membranfläche der Membrantrenneinheit kleiner als beim Stand der Technik bei gleicher Trennleistung der Membrantrenneinheit.

Ein weiterer Vorteil des Gasseparators besteht darin, dass viele handelsübliche Membranmodule für Reaktionsgemische mit gelösten und/oder ungelösten Gasanteilen ungeeignet sind, da diese nicht für eine ausreichende permeatseitige Gasabfuhr ausgelegt sind und je nach Menge des permeatseitig abzuführenden Gasvolumenstroms permeatseitig einen Gegendruck aufbauen, welcher die Flüssigpermeatleistung reduziert oder sogar zu einer Zerstörung der Membran führen kann.

Um eine Desaktivierung des Katalysators und eine Beschädigung der Membran durch Entgasung zu vermeiden, sowie einen verbesserten Membranrückhalt zu erreichen, war es bei herkömmlichen Anlagen üblich, das Permeat der Membrantrenneinheit zu entgasen. Derartige Maßnahmen sind dank des erfindungsgemäßen Gasseparators nicht mehr zwingend erforderlich, da bereits vor der Membrantrenneinheit schädliche Gasanteile entfernt werden. Im Gegensatz zum Stand der Technik erfolgt die Entgasung erfindungsgemäß feedseitig und nicht permeatseitig. Eine permeatseitige Nachentgasung kann jedoch trotz vorhergehender Entgasung notwendig sein, wenn Homogenkatalysator während der Membranabtrennung mit gelöstem Gas stabilisiert werden muss.

Eine besondere Weiterbildung der Erfindung sieht vor, dass im äußeren Flüssigkeitsumlauf, stromaufwärts vor der Membrantrenneinheit, ein Wärmetauscher zum Kühlen des Feeds der Membrantrenneinheit angeordnet ist, und dass der Gasseparator stromabwärts hinter dem Wärmetauscher vor der Membrantrenneinheit angeordnet ist. Ein solcher Wärmetauscher dient zum Abführen der Reaktionswärme exothermer Reaktionen. Es macht Sinn, den Gasseparator hinter dem Wärmetauscher anzuordnen, da der Flüssigkeitsumlauf dann eine niedrigere Temperatur hat, sodass er sich besser entgasen lässt. Außerdem führen höhere Temperaturen bei vielen Membranmaterialien zu einer Reduzierung des Membranrückhaltes und damit zu einer Verschlechterung der Abtrennung.

Die Pumpe zur Bewegung des äußeren Flüssigkeitsumlaufs wird vorzugsweise ebenfalls stromaufwärts vor der Membrantrenneinheit angeordnet. Insbesondere wird sie auch stromaufwärts vor dem Gasseparator und - sofern eine Wärmetauscher im Flüssigkeitsumlauf vorgesehen ist - stromaufwärts vor dem Wärmetauscher angeordnet.

Eine bevorzugte Weiterbildung der Erfindung sieht vor, dass die Membrantrenneinheit als mehrstufige Membrankaskade ausgeführt ist. Dadurch lässt sich die Permeatqualität verbessern und/oder die Membranfläche verkleinern.

Als Membrankaskade kommt dabei eine Tannenbaumverschaltung oder ein Feed-und-Bleed-System, wie insbesondere eine Verstärkerkaskade oder eine Abtriebskaskade in Betracht.

Einfachstenfalls wird die Membrantrenneinheit als "Feed-and-Bleed"-System mit einem einzelnen Rezirkulationskreislauf (einem so genannten Loop) ausgeführt. Im Rezirkulationskreislauf wird ein Teil des Retentats in den Feed zurückgeführt.

Die Permeatqualität kann zunächst dadurch verbessert werden, dass die Membrantrenneinheit aus mehreren seriellen Loops aufgebaut wird.

Weiter kann die Permeatqualität dadurch verbessert werden, dass ein mehrstufiges Feed-and-Bleed-System als Membrantrenneinheit genutzt wird. Dabei handelt es sich um eine mehrstufige Membrankaskade, welche über mehrere Rezirkulationskreisläufe bzw. Loops verfügt. Kaskadierte Feed-and-Bleed-Membransysteme können entweder als "Abtriebskaskade" oder als "Verstärkerkaskade" ausgeführt sein. Jede Stufe einer solchen Kaskade kann aus einen oder mehreren Loops aufgebaut werden.

Gegenüber der in den Membrantechnik gebräuchlichen und daher bekannten "Tannenbaumverschaltung" (Melin/Rautenbach) erlauben als "Feed und Bleed" System aufgebaute Membrankaskaden den Betrieb bei in Qualität und/oder Quantität schwankender Zulaufbedingungen und/oder sich zeitlich verändernder Membranperformance. Bei hohen Konzentrierungsfaktoren führt eine Abtriebskaskade zu einer besseren Gesamtpermeatqualität als eine Verstärkungskaskade bei gleicher verbauter Membranfläche. Weiterhin besitzt eine Verstärkungskaskade den Nachteil, dass benötigte Fläche gegenüber einer einstufigen Membrantrenneinheit mehr als doppelt so groß ist. Bei einer Abtriebskaskade hingegen können auch nahezu beliebige Membranfläche zwischen einer einstufigen Membrantrenneinheit und einer Verstärkungskaskade umgesetzt werden. Dies ist insbesondere dann wichtig, wenn eine Verstärkungskaskade aufgrund der hohen benötigten Membranfläche nicht wirtschaftlich ist und eine einstufige Membrantrenneinheit aufgrund unzureichender Abtrennung nicht einsetzbar ist.

Unter diesen Voraussetzungen bietet es sich an, eine zweistufige Membrankaskade mit teilweiser Permeatrückführung als Membrantrenneinheit einzusetzen. Zurückgeführt wird dabei das Permeat aus dem Rezirkulationskreislauf bzw. den Kreisläufen mit der schlechtesten Permeatqualität, welches in der Regel die Loops mit der höchsten Retentatkonzentration am Ende der Aufkonzentrierungsstrecke sind. Diese Verschaltung wird in der Membrantechnik "zweistufige Abtriebskaskade" genannt. Die Rezirkulationskreisläufe mit Permeatrückführung am Ende der Kaskade werden auch als Konzentratloops bezeichnet. Eine Verschaltung mit Konzentratloops mit Permeatrückführung ermöglicht ein reineres Gesamtpermeat.

Ganz besonders bevorzugt wird die genutzte Membranfläche der Konzentratloops kleiner ausgeführt als die genutzte Membranfläche der übrigen Loops. Auf diese Weise wird ohne Verschlechterung des Trennergebnisses der Membranflächenbedarf gesenkt.

Alternativ wird die Membrantrenneinheit wird als zweistufige Verstärkungskaskade ausgeführt. Bei einer zweistufigen Verstärkungskaskade handelt es sich um eine mehrstufige Membrankaskade mit teilweiser Retentatrückführung. Zurückgeführt wird dabei das Gesamtretentat aus der zweiten Stufe. Sowohl die erste als auch die zweite Stufe der zweistufigen Verstärkungskaskade können aus einem oder mehreren Membranloops aufgebaut werden. Zum einen sind die hier benötigten Konzentrierungsfaktoren niedrig genug, dass eine Verstärkungskaskade aufgrund des Trennergebnisses gegenüber einer Abtriebskaskade vorteilhaft ist. Zum anderen sind die zu erzeugenden Permeatmengen und damit die benötigen Membranflächen so klein, dass eine Verstärkungskaskade wirtschaftlich ist.

Eine ganz besonders bevorzugte Ausführungsform der Erfindung sieht vor, dass zwischen Gasseparator und Strahlschlaufenreaktor kein Förderorgan vorgesehen ist, dergestalt, dass der Strahlschlaufenreaktor das von dem Gasseparator aus dem äußeren Flüssigkeitsumlauf abgeschiedene Gas selbstständig ansaugt. Der starke innere Fluidstrom durch den Jet-Loop-Reaktor wirkt nämlich wie eine Wasserstrahlpumpe, sodass sich ohne zusätzliche Aggregate die im Gasseparator abgeschiedenen Gasanteile des äußeren Flüssigkeitsumlaufs in den Reaktor zurückführen lassen. Kein Förderorgan bedeutet in diesem Zusammenhang, dass kein unmittelbares Förderorgan wie eine Pumpe zwischen Gasseparator und Strahlschlaufenreaktor vorgesehen ist. Die Förderleistung wird letztendlich von der Pumpe erbracht, die den äußeren Flüssigkeitsumlauf und die Strömung in dem Reaktor erzeugt und folglich mittelbar den Wasserpumpeneffekt verursacht.

In einer weiteren bevorzugten Ausführungsform der Erfindung erstreckt sich in dem Strahlschlaufenreaktor ein rohrförmiger Reaktionsraum, in welchen eine Strahldüse zum Eindüsen der Flüssigkeit in den Reaktionsraum sowie ein Saugrohr zum Ansaugen des Gases gemeinsam münden, und an welchem ein von einer Prallplatte abgeschirmter Abzug für den äußeren Flüssigkeitsumlauf vorgesehen ist.

Die Strahldüse kann in dem sich vertikal erstreckenden Reaktor nach oben oder nach unten gerichtet sein. Die gemeinsame Mündung von Strahldüse und Saugrohr bewirkt eine intensive Vermischung der flüssigen und gasförmigen Reaktionskomponenten (Wasserpumpeneffekt). Das Gas kann über das Saugrohr von außen angesaugt werden oder aus einem Bereich innerhalb des Reaktionsraums, in welchem sich eine Gasglocke erstreckt. Der Abzug kann oben oder unten am Reaktor angeordnet sein. Die Abschirmung des Abzugs durch die Prallplatte verringert das Eintragen von Gasblasen aus dem inneren Flüssigkeitsumlauf in den äußeren Flüssigkeitsumlauf. Zur strömungsdynamischen Verbesserung kann mindestens ein Leitrohr, welches sich konzentrisch durch den Reaktionsraum erstreckt, vorgesehen werden. Dadurch wird die Vermischung der flüssigen und gasförmigen Phase intensiviert. Es können auch mehrere Leitrohre fluchtend hintereinander angeordnet sein. Durch das Leitrohr strömt dann die Reaktionsmischung in Hauptstromrichtung, wird am Ende des Leitrohrs umgelenkt und strömt außerhalb des Leitrohrs zurück. Das Leitrohr stellt eine bauliche Trennung der beiden Strömungsrichtungen der inneren Schlaufe dar.

Die erfindungsgemäße Vorrichtung eignet sich in hervorragender Weise zur homogenkatalytischen Umsetzung einer Flüssigkeit mit einem Gas und gegebenenfalls einem weiteren Fluid, bei welchem zumindest ein Zielprodukt der Umsetzung mit dem Permeat der Membrantrenneinheit aus dem Flüssigkeitsumlauf ausgetragen wird.

Gegenstand der Erfindung ist mithin auch ein Verfahren zur homogenkatalytischen Umsetzung einer Flüssigkeit mit einem Gas und gegebenenfalls einem weiteren Fluid, wobei die die Umsetzung in einer erfindungsgemäßen Vorrichtung durchgeführt wird, und wobei zumindest ein Zielprodukt der Umsetzung mit dem Permeat der Membrantrenneinheit aus dem Flüssigkeitsumlauf ausgetragen wird.

Dank des Gasseparators ist es möglich, einen Flüssigkeitsumlauf mit gasförmigem Anteil zu prozessieren. Dies ist bis zu einem Gasanteil von etwa 30 Vol% im Feed der Membrantrenneinheit möglich. Eine bevorzugte Weiterbildung des erfindungsgemäßen Verfahrens besteht mithin darin, dass der äußere Flüssigkeitsumlauf stromaufwärts vor der Membrantrenneinheit ein Gemisch darstellt, welches eine flüssige Phase und eine darin dispers verteilte gasförmige Phase umfasst, wobei Volumenanteil der gasförmigen Phase zwischen null und dreißig Prozent beträgt.

Der Gasseparator ermöglicht aber auch die Prozessierung größerer Gasmengen. Eine bevorzugte Weiterbildung des erfindungsgemäßen Verfahrens besteht mithin darin, dass der äußere Flüssigkeitsumlauf stromaufwärts vor dem Gasseparator ein Gemisch darstellt, welches eine flüssige Phase und eine darin dispers verteilte gasförmige Phase umfasst, wobei Volumenanteil der gasförmigen Phase zwischen dreißig und 100 Prozent beträgt. Der Gasseparator dient dann dazu, den höheren Gasanteil abzutrennen, sodass stromabwärts hinter dem Gasseparator, im Feed der Membrantrenneinheit, ein Gasgehalt unter 30 % Vol. vorliegt, der die Membran nicht schädigt.

Diese Reaktionen können zweiphasig (flüssig / gasförmig) oder dreiphasig (flüssig / flüssig / gasförmig bzw. flüssig / gasförmig / gasförmig) sein. Im Flüssigkeitsumlauf können auch geringe Feststoffanteile vorhanden sein.

Beispiele für die durchführbaren Reaktionen sind Oxidationen, Epoxidierungen, Hydroformylierungen, Hydroaminierungen, Hydroaminomethylierungen, Hydrocyanierungen, Hydrocarboxyalkylierung, Aminierungen, Ammonoxydation, Oximierungen, Hydrosilylierungen, Ethoxylierungen, Propoxylierungen, Carbonylierungen, Telomerisierungen, Methathesen, Suzuki-Couplings oder Hydrierungen.

Besonders bevorzugt ist die Vorrichtung zur Hydroformylierung, also zur Umsetzung von Verbindungen mit olefinischen Doppelbindungen mit Synthesegas zu Aldehyden und/oder Alkoholen geeignet.

Die Verwendung der beschriebenen Vorrichtung zur Durchführung der genannten Verfahren ist ebenfalls erfindungsgegenständlich.

Die erfindungsgemäße Vorrichtung kann unter anderem für die Umsetzung einer Flüssigkeit mit einem Gas eingesetzt werden, wobei sowohl das Gas als auch die Flüssigkeit mindestens einen Reaktanden aufweisen.

Die Reaktionsprodukte werden in der Flüssigphase mit dem Permeat ausgetragen.

In der erfindungsgemäßen Apparatur können Reaktionen im Druckbereich von 0.2 bis 40 MPa (absolut) und im Temperaturbereich von 0 bis 350 °C durchgeführt werden. Die Reaktion findet dabei bevorzugt an einem in der flüssigen Phase homogen gelösten Katalysator statt.

Bevorzugt werden in der erfindungsgemäßen Vorrichtung Reaktionen durchgeführt, bei denen der Katalysator mit dem flüssigen Einsatzstoff eingespeist wird und in der flüssigen Produkt/Edukt-Phase homogen gelöst ist, wie beispielsweise die Herstellung von Aldehyden und/oder Alkoholen durch Hydroformylierung von Verbindungen mit olefinischen Doppelbindungen in Gegenwart von Kobalt- oder Rhodiumcarbonylen mit oder ohne Zusatz von Phosphor enthaltenden Liganden.

Die Auswahl des geeigneten Membranwerkstoffs ist in Hinblick auf den abzuscheidenden Katalysatorkomplex zu treffen: Da die Durchlässigkeit einer Membran für die verschiedenen Bestandteile des zu trennenden Feeds letztendlich eine Funktion der Zeit ist (die Membran ist nicht absolut undurchlässig für einen Katalysator, seine Durchtrittsgeschwindigkeit ist vielmehr im Vergleich zu den anderen Reaktionsteilnehmer deutlich langsamer) ist die Membran so auszuwählen, dass sie den abzuscheidenden Katalysatorkomplex bevorzugt zurückhält.

In dem erfindungsgemäßen Verfahren können Membranen eingesetzt werden, die auf Grund von ihren chemischen oder physikalischen Eigenschaften geeignet sind, phosphororganischen Metall-Komplexkatalysator und/oder freien Organophosphor-Ligand vorzugsweise in einem Maße von zumindest 50 % zurückzuhalten.

Entsprechende Membranen gehören zu der Klasse der Nanofiltrations-Membranen. Der Begriff Nanofiltration wird auf Membrantrennverfahren angewendet, welche eine Trenngrenze bzw. Molecuar weight cut-off (MWCO) im Bereich von 150 g/mol bis zu über 1 nm besitzen. Die Größe der Trenngrenze (engl.: "molecuar weight cut-off" - MWCO) gibt die Molekül- oder Partikelgröße einer Komponente an, die einen Membranrückhalt von 90% besitzt.

Eine übliche Bestimmungsmethode für die Trenngrenze ist in Y.H. See Toh, X.X. Loh, K. Li, A. Bismarck, A.G. Livingston, In search of a standard method for the characterisation of organic solvent nanofiltration membranes, J. Membr. Sci., 291 (2007) 120-125 angegeben.

Der Membranrückhalt $R_i$ berechnet sich aus der feedseitigen Konzentration der betrachteten Komponente i an der Membran $x_{iF}$ und der pemeatseitigen Konzentration der betrachteten Komponente i an der Membran $x_{iP}$ wie folgt:

$$R_i = 1 - x_{iP} / x_{iF}$$

Bevorzugt sollten die Membranen für den erfindungsgemäßen Einsatz einen MWCO von kleiner 1000 g/mol aufweisen.

Eine weitere Vorraussetzung für die Verwendbarkeit der Membran besteht darin, dass die Membran stabil gegenüber allen in der Reaktionsmischung vorhandenen Verbindungen, insbesondere gegenüber den Lösemitteln sein muss. Als stabil werden auch Membranen angesehen, welche - etwa durch Quellen des Membranpolymers hervorgerufen - eine zeitliche Veränderung des MWCO und/oder der Permeabilität erfahren, jedoch über die Betriebsdauer die Trennaufgabe erfüllen. Darüber hinaus sollte der Membranwerkstoff die Reaktionstemperatur ertragen. Membranmaterialien, die Reaktionstemperatur stabil und performant sind, gestatten den Verzicht einer aufwändigen Temperaturregelung.

Vorzugsweise werden Membranen eingesetzt, die eine trennaktive Schicht aus einem Material, ausgewählt aus Celluloseacetat, Cellulosetriacetat, Cellulosenitrat, regenerierter Cellulose, Polyimiden, Polyamiden, Polyetheretherketonen, sulfonierten Polyetheretherketonen, aromatischen Polyamiden, Polyamidimiden, Polybenzimidazolen, Polybenzimidazolone, Polyacrylnitril, Polyarylethersulfone, Polyester, Polycarbonaten, Polytetrafluorethylen, Polyvinylidenfluorid, Polypropylen, Polydimethylsiloxan, Silicone, Polyphosphazene, Polyphenylsulfide, Polybenzimidazole, 6.6 Nylon, Polysulfone, Polyaniline, Polyurethane, Acrylonitril/Glycidylmethacrylat (PANGMA), Polytrimethylsilylpropyne, Polymethylpentyne, Polyvinyltrimethylsilan, alpha-Aluminiumoxide, Titaniumoxide, gamma-Aluminiumoxide, Polyphenylenoxid, Siliciumoxide, Zirconiumoxide, mit Silanen hydrophobisierte keramische Membranen, wie sie in DE10308111 beschrieben sind, Polymere mit intrinsischer Mikroporösität (PIM) wie PIM-1 und anderen, wie sie z. B. in EP0781166 und in "Membranes" von I. Cabasso, Encyclopedia of Polymer Sience and Technlogy, John Wiley and Sons, New York, 1987, beschrieben werden, aufweisen. Die oben genannten Stoffe können insbesondere in der trennaktiven Schicht ggf. durch Zugabe von Hilfsstoffen vernetzt vorliegen oder als so genannte Mixed Matrix Membranes mit Füllstoffen wie z. B. Carbon Nano Tubes, Metal Organic Frameworks oder Hollow Spheres sowie Partikel von anorganischen Oxiden oder anorganische Fasern, wie z. B. Keramik- oder Glasfasern versehen sein.

Besonders bevorzugt werden Membranen eingesetzt, die als trennaktive Schicht eine Polymerschicht aus Polydimethylsiloxan, Polyimid, Polyamidimid, Acrylonitril/Glycidylmethacrylat (PANGMA), Polyamid oder Polyetheretherketon aufweisen, die aus Polymeren mit intrinsischer Mikroporosität (PIM) wie PIM-1 aufgebaut sind, oder wobei die trennaktive Schicht über eine hydrophobierte keramische Membran aufgebaut ist. Ganz besonders bevorzugt werden Membranen aus Silikonen oder Polyamidimid eingesetzt. Solche Membranen sind kommerziell erhältlich.

Neben den oben genannten Materialien können die Membranen weitere Materialien aufweisen. Insbesondere können die Membranen Stütz- oder Trägermaterialien aufweisen, auf die die trennaktive Schicht aufgebracht ist. Bei solchen Verbundmembranen liegt neben der eigentlichen Membrane noch ein Stützmaterial vor. Eine Auswahl von Stützmaterialien beschreibt EP0781166, auf welches explizit verwiesen wird.

Eine Auswahl von kommerziell erhältlicher Nanofiltrationsmembranen sind die MPF und Selro Serien von Koch Membrane Systems, Inc., unterschiedliche Typen von Solsep BV, die Starmem™ Serie von Grace/UOP, die DuraMem™ und PuraMem™ Serie von Evonik Industries AG, die Nano-Pro Serie von Bio-Pure Technology, die HITK-T1 von IKTS, sowie oNF-1, oNF-2 und NC-1 von der GMT Membrantechnik GmbH.

Wenn Strahlschlaufenreaktor und Membrantrenneinheit in einem gemeinsamen äußeren Flüssigkeitsumlauf angeordnet sind, bedingt dies, dass der Durchsatz durch Reaktor und Membran gleich sein muss. Aus apparativen Gründen kann die Durchsatzleistung von Reaktor und Membran aber unterschiedlich sein. Um dann dennoch einen Flüssigkeitsumlauf oder ideale Membranüberströmung zu bewerkstelligen, ist es möglich, den Apparat mit der niedrigen Durchflussleistung mit einer Bypassleitung auszustatten, welche eine Teilumfahrung des hydraulischen Hindernisses ermöglicht. Dementsprechend weist die Vorrichtung mindestens eine Bypassleitung auf, welche im äußeren Flüssigkeitsumlauf parallel zum Strahlschlaufenreaktor oder zur Membrantrenneinheit angeordnet ist.

In einer weiteren bevorzugten Ausführungsform der Vorrichtung weist diese nicht nur einen Stahlschlaufenreaktor auf, sondern eine Vielzahl von parallel schaltbaren Strahlschlaufenreaktoren mit einem gemeinsamen äußeren Flüssigkeitsumlauf, wobei die Membrantrenneinheit innerhalb des gemeinsamen äußeren Flüssigkeitsumlaufs angeordnet ist. Eine Mehrzahl von spezifisch kleiner dimensionierten Strahlschlaufenreaktoren erlaubt durch Zu- und Abschalten einzelner Reaktoren eine flexible Anpassung der Umsatzleistung der Vorrichtung an die Bedarfslage. Dies ermöglicht eine wirtschaftliche Ausnutzung der Vorrichtung bei wechselnder Nachfrage.

Die Membrantrenneinheit kann dementsprechend auch parallelisiert ausgeführt werden: Durch Zu- und Abschalten von einzelnen, parallel geschalteten Membranmodulen kann die Gesamtmembranfläche der Membrantrenneinheit flexibel an die Anlagenkapazität angepasst werden. Eine bevorzugte Weiterbildung der Erfindung ist daher durch eine Membrantrenneinheit gekennzeichnet, welche eine Vielzahl von dergestalt parallel schaltbaren Membranen umfasst, dass die gesamte aktive Membranoberfläche der Membrantrenneinheit durch Zu- und Abschalten der Membranen einstellbar ist.

Die Erfindung soll nun anhand von Ausführungsbeispielen näher erläutert werden. Hierfür zeigen:

Figur 1: erfindungsgemäße Vorrichtung mit einfacher Membrantrenneinheit;

Figur 2: Detaildarstellung einer Membrantrenneinheit als zweistufige Abtriebskaskade;

Figur 3: Detaildarstellung zweite Membrantrenneinheit als zweistufige Verstärkungskaskade;

Figur 4: erfindungsgemäße Vorrichtung mit kaskadierter Membrantrenneinheit und destillativer Permeataufbereitung.

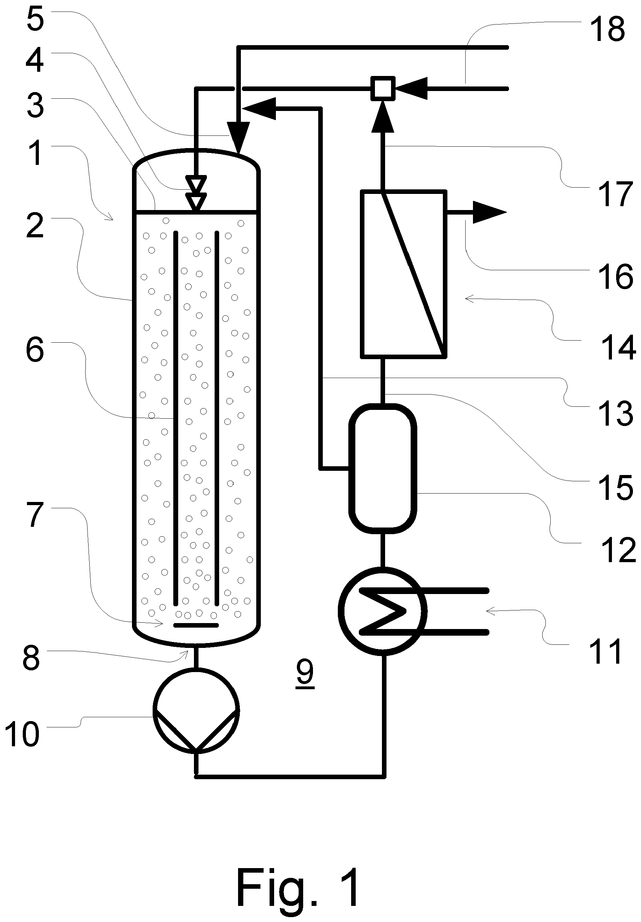

Figur 1 zeigt eine erste Ausführungsform der erfindungsgemäßen Vorrichtung mit einem Strahlschlaufenreaktor 1. Der Strahlschlaufenreaktor 1 umfasst einen rohrförmigen Reaktionsraum 2 in Gestalt eines Druckrohrs, der bis zu einem bestimmten Flüssigkeitsspiegel 3 mit flüssigem Reaktionsgemisch gefüllt ist. Oberhalb des Flüssigkeitsspiegels bildet sich eine Gasglocke aus gasförmigen Reaktionsteilnehmern aus. Aufgrund des Lösungsgleichgewichts sind die gasförmigen Reaktionsteilnehmer teilweise im flüssigen Reaktionsgemisch gelöst, teilweise befinden sich gasförmige Reaktionsteilnehmer als Gasphase in der Flüssigkeit (in der Zeichnung als Gasblasen dargestellt). Ebenfalls in der Reaktionsflüssigkeit gelöst ist der Homogen-Katalysator.

In das flüssige Reaktionsgemisch hinein ragt von oben herab eine Strahldüse 4, über welche flüssige Reaktionsteilnehmer mit hoher kinetischer Energie eingedüst werden. Gasförmige Reaktionsteilnehmer gelangen durch eine Gaszufuhr 5 in den Reaktionsraum 2. Der Stahldüse 4 baulich zugeordnet ist ein Saugrohr, welches das Gas aus dem mit Gas gefüllten Teil des Reaktionsraums 2 ansaugt und mit dem Fluidstrom vermischt. Hierzu sind die Mündungen von Saugrohr und Strahldüse eng benachbart und münden gemeinsam in den Reaktionsraum. Durch die hohe Strömungsgeschwindigkeit der aus der Strahldüse 4 austretenden flüssigen Reaktionsteilnehmer werden die gasförmigen Reaktionsteilnehmer aus der Gaszufuhr 5 kommend mitgerissen (vergleichbar mit einer Wasserstrahlpumpe).

Durch den Reaktionsraum 2 erstreckt sich ein Leitrohr 6, konzentrisch und koaxial zum Druckrohr. Das Leitrohr 6 dient dazu, innerhalb des Reaktionsraums 2 einen inneren Flüssigkeitsumlauf zu schaffen: Die eingedüste Reaktionsflüssigkeit strömt von der Strahldüse 4 herab durch das Leitrohr 6 und wird von dem am anderen Ende des Reaktionsraums 2 angeordnete Prallplatte 7 umgelenkt, sodass der Strom außerhalb des Leitrohrs 6 wieder nach oben strömt. Auf diese Weise entsteht innerhalb des Reaktionsraums 2 ein innerer Flüssigkeitsumlauf, in welchem die Reaktionspartner intensiv vermischt und umgesetzt werden.

Unterhalb der Prallplatte 7 ist ein Abzug 8 vorgesehen, durch welchen kontinuierlich Reaktionsgemisch aus dem Reaktionsraum 2 abgezogen und in einen äußeren Flüssigkeitsumlauf 9 eingespeist wird. Die Prallplatte 7 schirmt den Abzug 8 zum inneren Flüssigkeitsumlauf ab, sodass Gasblasen kaum in den äußeren Flüssigkeitsumlauf 9 geraten. Der äußere Flüssigkeitskreislauf besteht daher überwiegend aus flüssigen Reaktanden, gelöstem Katalysator und gelösten gasförmigen Reaktanden.

Für die Ausübung der Erfindung ist es unerheblich, ob Strahldüse 4 nach unten gerichtet und Prallplatte 7 unterhalb der Strahldüse 4 angeordnet ist. Es ist auch möglich, vom Boden des Reaktors her nach oben einzudüsen. Der Abzug kann in beiden Fällen oben oder unten im Reaktor angeordnet sein. Die Prallplatte ist entsprechend so anzuordnen, dass sie den Abzug abschirmt.

Der äußere Flüssigkeitsumlauf 9 wird von einer Pumpe 10 bewegt. Bei der Pumpe 10 handelt es sich um eine Peripheralradpumpe, welche auch flüssig/gasförmige Gemische zu fördern vermag. Geringfügige Gasblasen sind daher unschädlich.

Stromabwärts hinter der Pumpe 10 ist ein Wärmetauscher 11 angeordnet, mittels welchem abhängig von dem Reaktionstyp Wärme in den äußeren Flüssigkeitsumlauf 9 ein- oder ausgetragen werden kann. Darüber hinaus kann der Strahlschlaufenreaktor 1 selbst mit einem Wärmetauscher versehen sein, welcher den Reaktionsraum umgibt (nicht dargestellt). Bei der Durchführung von exothermen Reaktionen wie beispielsweise Hydroformylierungen, wird über den Wärmetauscher 11 die Reaktionswärme abgeführt. Der Wärmetauscher 11 kühlt dann den äußeren Flüssigkeitsumlauf 9.

Stromabwärts hinter dem Wärmetauscher 11 ist ein Gasseparator 12 angeordnet. Der Gasseparator 12 scheidet Gasanteile aus dem äußeren Flüssigkeitsumlauf 9 ab und führt diese dem Strahlschlaufenreaktor 1 zu. Zu diesem Zwecke ist der Gasseparator 12 über eine Gasleitung 13 mit der Gaszufuhr 5 des Strahlschlaufenreaktors 1 verbunden. In der Gasleitung 13 ist kein Förderorgan angeordnet, da der Strahlschlaufenreaktor das abgeschiedene Gas aus dem Gasseparator 12 selbstständig ansaugt.

Stromabwärts hinter dem Gasseparator 12 ist eine Membrantrenneinheit 14 angeordnet. Wie jede Membran, weist die Membrantrenneinheit 14 drei Anschlüsse auf, nämlich Feed 15, Permeat 16 und Retentat 17. Das über den Feed 15 anströmende Reaktionsgemisch wird an der Membran in Permeat 16 und Retentat 17 getrennt. Da die Membran für den gelösten Katalysatorkomplex weniger durchlässiger ist als für die übrigen Komponenten des Feeds 15, verbleibt der Katalysator diesseits der Membran und wird im Retentat 17 angereichert. Bezogen auf den Katalysator weist die Membrantrenneinheit 14 eine bessere Durchlässigkeit der Wertprodukte auf, sodass sich die Wertprodukte bezogen auf den Katalysator im Permeat 16 anreichern. Das Permeat 16 wird weiter zur Aufbereitung geführt (in Figur 1 nicht dargestellt); das katalysatorreiche Retentat 17 wird mit frischem, flüssigen Edukt 18 vermischt über die Strahldüse 4 in den Reaktor 1 zurückgeführt.

Der erfindungsgemäß vor der Membrantrenneinheit 14 angeordnete Gasseparator 12 bewirkt eine Verringerung des Volumenstroms des Feeds 15 der Membrantrenneinheit 14, da Gasblasen und gelöstes Gas aus dem Flüssigkeitsumlauf 9 abgeschieden und über die Gasleitung 13 direkt in den Strahlschlaufenreaktor 1 zurückgeführt werden. Aufgrund des verringerten Feedvolumenstroms kann die Membranfläche der Membrantrenneinheit 14 bei gleicher Abtrennungsleistung kleiner ausfallen als bei herkömmlichen Anlagen ohne Gasseparator 12. Insbesondere die Durchführung von homogen katalysierten Hydroformylierungen wird dadurch deutlich wirtschaftlicher, da die Abscheidung von dabei eingesetzten Rhodium-Phosphit-Komplexkatalysatoren kostspielige Membranen erfordert.

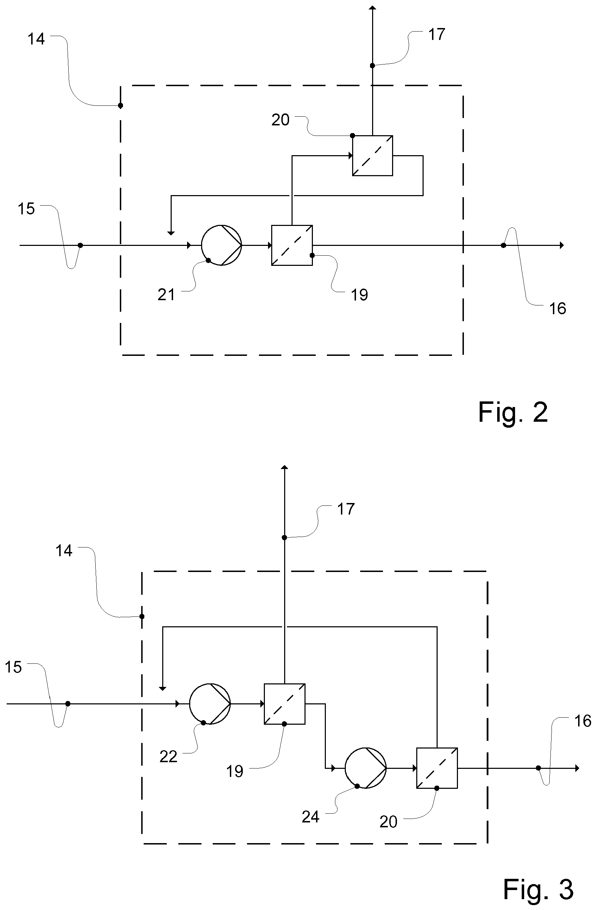

In Figur 1 wurde die Membrantrenneinheit 14 in ihrer einfachsten Bauform mit nur einer Membran dargestellt. In der Praxis wird die Membrantrenneinheit 14 vielmehr als mehrstufige Membrankaskade ausgeführt werden, wie sie die Figuren 2 und 3 zeigen.

Figur 2 zeigt den prinzipiellen Aufbau einer als eine zweistufige Abtriebskaskade mit zwei Stufen 19, 20 ausgeführten Membrantrenneinheit 14. Der ersten Stufe 19 wird der entgaste Feed 15 aufgegeben. Das Permeat der ersten Stufe 19 entspricht dem resultierenden Permeat 16 der Membrantrenneinheit 14. Das Retentat der ersten Stufe 19 wird ohne eine weitere Druckerhöhung der zweiten Stufe 20 aufgegeben. Das Retentat der zweiten Stufe 20 entspricht dem resultierenden Retentat 17 der Membrantrenneinheit 14. Das Permeat der zweiten Stufe 20 wird mit dem Feed 15 der Membrantrenneinheit 14 vermischt und über eine interne Pumpe 21 wieder der ersten Stufe 19 zugeführt. Das Permeat der zweiten Stufe 20 entspricht somit der internen Permeatrückführung der als Abtriebskaskade ausgeführten Membrantrenneinheit 14.

Figur 3 zeigt den prinzipiellen Aufbau einer als eine zweistufige Verstärkungskaskade mit zwei Stufen 19, 20 ausgeführten Membrantrenneinheit 14. Der ersten Stufe 19 wird der entgaste Feed 15 aufgegeben. Das Retentat der ersten Stufe 19 entspricht dem resultierenden Retentat 17 der Membrantrenneinheit 14 und verlässt diese. Das Permeat der ersten Stufe 19 wird mittels einer zweiten Druckerhöhungspumpe 24 wieder auf Druck gebracht, um den Transmembrandruck der ersten Stufe 19 wieder auszugleichen. Sodann erfolgt die zweiten Stufe 20 der Membrantrennung. Das dabei anfallende Permeat entspricht dem resultierenden Permeat 16 der gesamten Membrantrenneinheit 14. Das Retentat der zweiten Stufe 20 wird mit dem Feed 15 vermischt und über eine erste Druckerhöhungspumpe 22 in die erste Stufe 19 zurückgeführt. Das Retentat der zweiten Stufe 20 stellt somit die interne Retentatrückführung der Verstärkungskaskade dar.

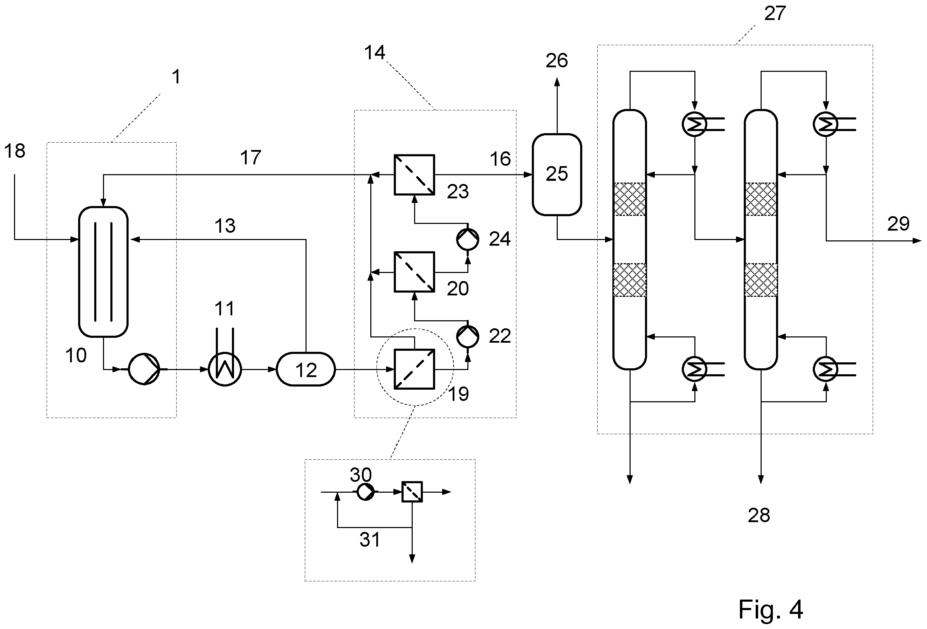

Figur 4 zeigt eine erfindungsgemäße Vorrichtung, bei der die Membrantrenneinheit 14 drei Membranstufen 19, 20, 23 aufweist, die in Richtung ihrer Permeate hintereinander geschaltet sind. Zwei Drückerhöhungspumpen 22, 24 gleichen die an den Stufen 19, 20 abgefallenen Transmembrandrücke aus. Die jeweiligen Retentate der Stufen 19, 20, 23 werden als resultierendes Retenat 17 vereinigt und zum Strahlschlaufenreaktor 1 rezykliert. Jede Stufe 19, 20, 23 umfasst auch eine eigene Stufenpumpe 30 und einen internen Loop 31, wie in der Ausschnittsvergrößerung der ersten Stufe 19 dargestellt.

Das Permeat der dritten Stufe 23 bildet das resultierende Permeat 16 der Membrantrenneinheit 14. Es wird in einem Nachentgaser 25 von restlichen Gasbestandteilen entgast, die etwa zur Stabilisierung des Katalysatorkomplexes während der Katalysatorabtrennung über die drei Stufen 19, 20, 23 von dem Gasseparator 12 nicht abgetrennt wurden. Aufgrund des inzwischen eingetretenen Druckverlustes kann das im Nachentgaser 25 abgetrennte Gas nicht ohne Kompression in den Strahlschlaufenreaktor 1 zurückgeführt werden und wird deswegen der Einfachheit halber als Abgas 26 verworfen.

Das im Gasseparator 12 abgetrennte Gas hat indes noch keine Membran gesehen und kann daher ohne Förderorgan über die Gasleitung 13 zurück in den Strahlschlaufenreaktor 1 rezykliert werden. Dort wird es getrennt von dem gasförmigen Edukt 18 eingespeist, also nicht über eine gemeinsame Gaszufuhr.

In Figur 4 ist noch eine destillative Aufarbeitung 27 des nachentgasten Permeats 16 dargestellt, im Zuge derer die eigentlichen Zielprodukte 28, 29 gewonnen werden. Bei den Zielprodukten handelt es sich um n-Butanal 28 bzw. iso-Butanal 29, was durch Hydroformylierung von Propen mit Kohlenmonoxid und Wasserstoff (Synthesegas), alles Edukt 18, gewonnen wurde. Bei dem im Gasseparator 12 abgeschiedenen Gas handelt es sich im Wesentlichen um nicht umgesetztes Synthesegas.

## Bezugszeichenliste

| 1 | Strahlschlaufenreaktor |
|---|---|
| 2 | Reaktionsraum |
| 3 | Flüssigkeitsspiegel |
| 4 | Strahldüse |
| 5 | Gaszufuhr |
| 6 | Leitrohr |
| 7 | Prallplatte |
| 8 | Abzug |
| 9 | äußerer Flüssigkeitsumlauf |
| 10 | Pumpe |
| 11 | Wärmetauscher |
| 12 | Gasseparator |
| 13 | Gasleitung |
| 14 | Membrantrenneinheit |
| 15 | Feed |
| 16 | Permeat |
| 17 | Retentat |
| 18 | Edukt |
| 19 | erste Stufe |
| 20 | zweite Stufe |
| 21 | Pumpe innerhalb der Membrantrenneinheit |
| 22 | (erste) Druckerhöhungspumpe |
| 23 | dritte Stufe |
| 24 | zweite Druckerhöhungspumpe |
| 25 | Nachentgaser |
| 26 | Abgas |
| 27 | destillative Aufarbeitung |
| 28 | erstes Zielprodukt |
| 29 | zweites Zielprodukt |
| 30 | Stufenpumpe |

31 Loop

**Patentansprüche**

1. Vorrichtung für die kontinuierliche, homogenkatalytische Umsetzung einer Flüssigkeit mit einem Gas und gegebenenfalls einem weiteren Fluid, wobei die Vorrichtung mindestens einen Strahlschlaufenreaktor mit einem von mindestens einer Pumpe getriebenen, äußeren Flüssigkeitsumlauf umfasst, und wobei die Vorrichtung mindestens eine den Homogenkatalysator bevorzugt zurückhaltende Membrantrenneinheit aufweist, welche in dem äußeren Flüssigkeitsumlauf des Strahlschlaufenreaktors angeordnet ist, **gekennzeichnet durch** einen im äußeren Flüssigkeitsumlauf des Strahlschlaufenreaktors angeordneten Gasseparator, welcher dafür eingerichtet ist, Gas aus dem äußeren Flüssigkeitsumlauf abzuscheiden und in den Strahlschlaufenreaktor zurückzuführen, und **dadurch gekennzeichnet, dass** im äußeren Flüssigkeitsumlauf, stromaufwärts vor der Membrantrenneinheit, ein Wärmetauscher zum Kühlen des Feeds der Membrantrenneinheit angeordnet ist, und dass der Gasseparator stromabwärts hinter dem Wärmetauscher vor der Membrantrenneinheit angeordnet ist.

2. Vorrichtung nach Anspruch 1, **dadurch gekennzeichnet, dass** die Pumpe stromaufwärts vor der Membrantrenneinheit angeordnet ist, insbesondere, dass sie stromaufwärts vor dem Gasseparator und ganz besonders bevorzugt stromaufwärts vor dem Wärmetauscher angeordnet ist.

3. Vorrichtung nach Anspruch 1 oder 2, **dadurch gekennzeichnet, dass** die Membrantrenneinheit als mehrstufige Membrankaskade ausgeführt ist.

4. Vorrichtung nach einem der vorhergehenden Ansprüche, **dadurch gekennzeichnet, dass** zwischen Gasseparator und Strahlschlaufenreaktor kein Förderorgan vorgesehen ist, dergestalt, dass der Strahlschlaufenreaktor das aus dem äußeren Flüssigkeitsumlauf abgeschiedene Gas aus dem Gasseparator selbstständig ansaugt.

5. Vorrichtung nach einem der vorhergehenden Ansprüche, **dadurch gekennzeichnet, dass** sich in dem Strahlschlaufenreaktor ein rohrförmiger Reaktionsraum erstreckt, in welchen eine Strahldüse zum Eindüsen der Flüssigkeit in den Reaktionsraum sowie ein Saugrohr zum Ansaugen des Gases gemeinsam münden, und an welchem ein von einer Prallplatte abgeschirmter Abzug für den äußeren Flüssigkeitsumlauf vorgesehen ist.

6. Verfahren zur homogenkatalytischen Umsetzung einer Flüssigkeit mit einem Gas und gegebenenfalls einem weiteren Fluid, **dadurch gekennzeichnet, dass** die Umsetzung in einer Vorrichtung gemäß einem der Ansprüche 1 bis 5 durchgeführt wird, und dass zumindest ein Zielprodukt der Umsetzung mit dem Permeat der Membrantrenneinheit aus dem Flüssigkeitsumlauf ausgetragen wird.

7. Verfahren nach Anspruch 6, **dadurch gekennzeichnet, dass** der äußere Flüssigkeitsumlauf stromaufwärts vor der Membrantrenneinheit ein Gemisch darstellt, welches eine flüssige Phase und eine darin dispers verteilte gasförmige Phase umfasst, wobei Volumenanteil der gasförmigen Phase zwischen null und dreißig Prozent beträgt.

8. Verfahren nach Anspruch 1, **dadurch gekennzeichnet, dass** der äußere Flüssigkeitsumlauf stromaufwärts vor dem Gasseparator ein Gemisch darstellt, welches eine flüssige Phase und eine darin dispers verteilte gasförmige Phase umfasst, wobei Volumenanteil der gasförmigen Phase zwischen dreißig und 100 Prozent beträgt.

9. Verfahren nach Anspruch 6, 7 oder 8, **dadurch gekennzeichnet, dass** in der Vorrichtung Oxidationen, Epoxidierungen, Hydroformylierungen, Hydroaminierungen, Hydroaminomethylierungen, Hydrocyanierungen, Hydrocarboxyalkylierung, Aminierungen, Ammonoxidation, Oximierungen, Hydrosilylierungen, Ethoxylierungen, Propoxylierungen, Carbonylierungen, Telomerisierungen, Methathesen, Suzuki-Couplings oder Hydrierungen durchgeführt werden.

10. Verfahren nach Anspruch 9, **dadurch gekennzeichnet, dass** Verbindungen mit olefinischen Doppelbindungen durch Hydroformylierung mit Synthesegas zu Aldehyden und/oder Alkoholen umgesetzt werden.

11. Verwendung einer Vorrichtung nach einem der Ansprüche 1 bis 5 zur Durchführung homogenkatalytischer Umsetzungen, insbesondere zur Hydroformylierung.

**Claims**

1. Device for the continuous homogeneous catalytic reaction of a liquid with a gas and optionally a further fluid, wherein the device comprises at least one jet loop reactor having an external liquid circuit driven by at least one pump, and wherein the device has at least one membrane separation unit, preferably retaining the homogeneous catalyst, which membrane separation unit is arranged in the external liquid circuit of the jet loop reactor,
**characterized by**
a gas separator arranged in the external liquid circuit of the jet loop reactor, which gas separator is installed for separating off gas from the external liquid circuit and feeding it back into the jet loop reactor, and **characterized in that**,
in the external liquid circuit, upstream of the membrane separation unit, a heat exchanger is arranged for cooling the feed of the membrane separation unit, and **in that** the gas separator is arranged downstream of the heat exchanger ahead of the membrane separation unit.

2. Device according to Claim 1,
**characterized in that**
the pump is arranged upstream of the membrane separation unit, in particular **in that** it is arranged upstream of the gas separator and very particularly preferably upstream of the heat exchanger.

3. Device according to Claim 1 or 2,
**characterized in that**
the membrane separation unit is constructed as a multistage membrane cascade.

4. Device according to any of the preceding claims,
**characterized in that**
no transport element is provided between the gas separator and jet loop reactor, in such a manner that the jet loop reactor takes in independently from the gas separator gas separated off from the external liquid circuit.

5. Device according to any of the preceding claims,
**characterized in that**
a tubular reaction space extends in the jet loop reactor, into which a jet nozzle for injecting the liquid into the reaction space opens out and also a suction tube for intake of gas opens out conjointly, and at which a baffle plate-shielded take-off for the external liquid circuit is provided.

6. Method for the homogeneous catalytic reaction of a liquid with a gas and optionally a further fluid,
**characterized in that**
the reaction is carried out in a device according to any one of Claims 1 to 5, and **in that** at least one target product of the reaction is discharged from the liquid circuit with the permeate of the membrane separation unit.

7. Method according to Claim 6,
**characterized in that**
the external liquid circuit, upstream of the membrane separation unit is a mixture which comprises a liquid phase and a gaseous phase distributed dispersely therein, wherein volume fraction of the gaseous phase is between zero and thirty percent.

8. Method according to Claim 1,
**characterized in that**
the external liquid circuit, upstream of the gas separator, is a mixture which comprises a liquid phase and a gaseous phase distributed dispersely therein, wherein volume fraction of the gaseous phase is between thirty and 100 percent.

9. Method according to Claim 6, 7 or 8,
**characterized in that**
oxidations, epoxidations, hydroformylations, hydroaminations, hydroaminomethylations, hydrocyanations, hydrocarboxyalkylation, aminations, ammoxidation, oximations, hydrosilylations, ethoxylations, propoxylations, carbonylations, telomerizations, metatheses, Suzuki couplings or hydrogenations are carried out in the device.

10. Method according to Claim 9,
**characterized in that**
compounds having olefinic double bonds are reacted by hydroformylation with synthesis gas to form aldehydes and/or alcohols.

11. Use of a device according to any one of Claims 1 to 5 for carrying out homogeneous catalytic reactions, in particular for hydroformylation.

**Revendications**

1. Dispositif pour la conversion continue catalytique homogène d'un liquide avec un gaz et éventuellement un autre fluide, dans lequel le dispositif comprend au moins un réacteur à boucle à jet avec un circuit de liquide extérieur propulsé par au moins une pompe, et dans lequel le dispositif présente au moins une unité de séparation à membrane retenant de préférence le catalyseur homogène, qui est disposée dans le circuit de liquide extérieur du réacteur à boucle à jet, **caractérisé par** un séparateur de gaz disposé dans le circuit de liquide extérieur du réacteur à boucle à jet, qui est conçu pour séparer du gaz hors du circuit de liquide extérieur et le renvoyer dans le réacteur à boucle à jet, et **caractérisé en ce qu'**un échangeur de chaleur est disposé dans le circuit de liquide extérieur, en amont avant l'unité de séparation à membrane, pour le refroidissement de la charge de l'unité de séparation à membrane, et **en ce que** le séparateur de gaz est disposé en aval après l'échangeur de chaleur avant l'unité de séparation à membrane.

**2.** Dispositif selon la revendication 1, **caractérisé en ce que** la pompe est disposée en amont avant l'unité de séparation à membrane, en particulier **en ce qu'**elle est disposée en amont avant le séparateur de gaz et de préférence encore en amont avant l'échangeur de chaleur.

**3.** Dispositif selon une revendication 1 ou 2, **caractérisé en ce que** l'unité de séparation à membrane est formée par une cascade de membranes à plusieurs étages.

**4.** Dispositif selon l'une quelconque des revendications précédentes, **caractérisé en ce qu'**il n'est prévu aucun organe de transport entre le séparateur de gaz et le réacteur à boucle à jet, de telle manière que le réacteur à boucle à jet aspire automatiquement à partir du séparateur de gaz le gaz séparé hors du circuit de liquide extérieur.

**5.** Dispositif selon l'une quelconque des revendications précédentes, **caractérisé en ce qu'**une chambre de réaction tubulaire s'étend dans le réacteur à boucle à jet, dans laquelle débouchent en même temps une buse d'injection pour injecter le liquide dans la chambre de réaction ainsi qu'un tube d'aspiration pour aspirer le gaz, et sur laquelle il est prévu une évacuation protégée par une plaque d'impact pour le circuit de liquide extérieur.

**6.** Procédé pour la conversion catalytique homogène d'un liquide avec un gaz et éventuellement un autre fluide, **caractérisé en ce que** l'on effectue la conversion dans un dispositif selon l'une quelconque des revendications 1 à 5, et **en ce qu'**au moins un produit cible de la conversion est extrait hors du circuit de liquide avec le perméat de l'unité de séparation à membrane.

**7.** Procédé selon la revendication 6, **caractérisé en ce que** le circuit de liquide extérieur représente en amont avant l'unité de séparation à membrane un mélange, qui comprend une phase liquide et une phase gazeuse dispersée dans celle-ci, dans lequel la proportion volumique de la phase gazeuse vaut entre zéro et trente pour cent.

**8.** Procédé selon la revendication 1, **caractérisé en ce que** le circuit de liquide extérieur représente en amont avant le séparateur de gaz un mélange, qui comprend une phase liquide et une phase gazeuse dispersée dans celle-ci, dans lequel la proportion volumique de la phase gazeuse vaut entre trente et 100 pour cent.

**9.** Procédé selon une revendication 6, 7 ou 8, **caractérisé en ce que** l'on effectue dans le dispositif des oxydations, des époxydations, des hydroformylations, des hydroaminations, des hydroaminométhylations, des hydrocyanurations, une hydrocarboxyalkylation, des aminations, une ammonoxydation, des oximations, des hydrosilylations, des éthoxylations, des propoxylations, des carbonylations, des télomérisations, des métathèses, des couplages de Suzuki ou des hydrogénations.

**10.** Procédé selon la revendication 9, **caractérisé en ce que** l'on convertit des composés avec des doubles liaisons oléfiniques par hydroformylation avec un gaz de synthèse en aldéhydes et/ou en alcools.

**11.** Utilisation d'un dispositif selon l'une quelconque des revendications 1 à 5 pour la mise en œuvre de conversions catalytiques homogènes, en particulier pour l'hydroformylation.

# Fig. 1

Fig. 2

Fig. 3

Fig. 4

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- EP 1931472 B1
- WO 2010023018 A1
- DE 102012202779
- WO 2013034690 A1
- DE 10308111
- EP 0781166 A

### In der Beschreibung aufgeführte Nicht-Patentliteratur

- Bubble Columns. **P. ZEHNER ; M. KRAUSE.** Ullmann's Encyclopedia of Industrial Chemistry, Electronic Release. Wiley-VCH, 2005
- **ROY L.** Hydroformylation. Advances in Organometallic Chemistry. Springer Verlag, 1979, vol. 17, 1-60
- **B. CORNILS ; W. A. HERRMANN.** Applied Homogeneous Catalysis with Organometallic Compounds. VCH, 1996, vol. 1, 2
- **R. FRANKE ; D. SELENT ; A. BÖRNER.** Applied Hydroformylation. *Chem. Rev.,* 2012
- Grundlagen der Modul- und Anlagenauslegung. Springer, 2004
- **PRISKE, M. et al.** Reaction integrated separation of homogeneous catalysts in the hydroformylation of higher olefins by means of organophilic nanofiltration. *Journal of Membrane Science,* 15. September 2010, vol. 360 (1-2), 77-83
- **JANSSEN, M. ; WILTING, J. ; MÜLLER, C. ; VOGT, D.** Continuous Rhodium-Catalyzed Hydroformylation of 1-Octene with Polyhedral Oligomeric Silsesquioxanes (POSS) Enlarged Triphenylphosphine. *Angewandte Chemie International Edition,* 2010, vol. 49, 7738-7741
- **TOH, X.X. LOH ; K. LI ; A. BISMARCK ; A.G. LIVINGSTON.** search of a standard method for the characterisation of organic solvent nanofiltration membranes. *J. Membr. Sci.,* 2007, vol. 291, 120-125
- Membranes. **I. CABASSO.** Encyclopedia of Polymer Sience and Technlogy. John Wiley and Sons, 1987